(19) Europäisches Patentamt
European Patent Office
Office européen des brevets

(11) **EP 4 483 784 A1**

(12) **EUROPEAN PATENT APPLICATION**

(43) Date of publication:
**01.01.2025 Bulletin 2025/01**

(21) Application number: 23182105.9

(22) Date of filing: **28.06.2023**

(51) International Patent Classification (IPC):
*A61B 5/00* (2006.01)  *A61B 6/03* (2006.01)
*A61B 5/055* (2006.01)  *A61B 5/024* (2006.01)
*A61B 5/113* (2006.01)  *F21W 131/20* (2006.01)

(52) Cooperative Patent Classification (CPC):
**A61B 5/055; A61B 5/0077; A61B 6/44;**
A61B 5/02416; A61B 5/1135; A61B 6/032;
A61B 6/037; A61B 6/541

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **WEISS, Steffen**
  Eindhoven (NL)
• **SENEGAS, Julien Thomas**
  Eindhoven (NL)
• **WIRTZ, Daniel**
  5656AG Eindhoven (NL)

(74) Representative: **Philips Intellectual Property &
Standards
High Tech Campus 52
5656 AG Eindhoven (NL)**

(54) **LIGHTING FOR A MEDICAL IMAGING SYSTEM**

(57) A system comprising a medical imaging unit having an imaging zone defined by an interior region of a bore, and further comprising a lighting apparatus comprising one or more light output areas disposed within the bore. In one set of embodiments, there are provided a plurality of light output areas, distributed at different positions along an axial direction of the bore .In some embodiments, a controller is included to control a light output setting of each of the plurality of light output areas so as to form a controlled spatial light distribution from a composite of the light fields of the plurality of light output areas. In some embodiments, an x-ray input/output window is replaced with an x-ray transmissive and light diffusive panel for performing dual functions of permitting x-ray communication between an imaging apparatus and the imaging zone and forming at least one wide-area diffuse/homogenous light output area.

FIG. 2

**Description**

FIELD OF THE INVENTION

The present invention relates to the field of medical imaging systems.

BACKGROUND OF THE INVENTION

Various medical imaging modalities involve a patient's body being positioned within an imaging zone, for instance defined by an interior of a bore. Such imaging modalities include for example computed tomography (CT) imaging, magnetic resonance (MR) imaging, positron emission tomography (PET) and single-photon emission computed tomography (SPECT). These imaging modalities allow for detailed visualization of the body's internal structures and functions, aiding in accurate diagnosis and treatment planning.

In some imaging procedures, it is necessary to acquire images while the patient is in a certain respiratory phase or state, or when certain physiological parameters meet defined criteria. For example, abdominal and cardiac CT imaging is usually performed in short breath-holds to avoid any loss of image quality due to respiratory motion. Similar procedures are also performed in MRI imaging.

State-of-the-art CT and MRI imaging systems do not monitor the respiratory state of the patient, for example to track whether and when the patient is in a breath-hold state.

One approach to tracking vital parameters such as respiratory and cardiac rhythm would be to use a physical sensor attached to the patient. This has been demonstrated for a range of medical applications.

An approach which may be preferable is to use camera-based tracking of vital parameters such as respiratory and cardiac rhythm. Some such systems exist in the art and, in particular, can monitor the respiratory state of a patient in an MR system with a camera. The system may also trigger MR acquisition as soon as the patient starts a breath-hold. This is known as respiratory gating. At least one wide-angle camera is positioned at an end of the bore to image the patient. Similar principle might also be applied to CT imaging. Tracking of other physiological parameters might also be of value in assisting medical imaging procedures.

In addition to tracking respiration, camera imagery can also usefully be used to detect motion of the patient, as a means for detecting possible motion artefacts in the image data.

Thus, the use of cameras for motion monitoring during medical imaging has technical application. In such a context, the general aim is to derive a measure of motion of the body part that is currently in the image acquisition zone (e.g. within the bore of the scanner). In a simplest case, this may be used to indicate to an operator which of multiple acquired slices of a CT dataset may have image quality impaired by motion. In some cases, the motion signal might be used to trigger cessation of the imaging procedure.

Respiratory monitoring involves the detection of sub-millimeter motion of the abdomen and chest. It's application in CT imaging in particular is demanding because CT acquisition is frequently performed with a moving patient table.

SUMMARY OF THE INVENTION

One significant difficulty in detection of motion from camera images is the problem of intensity variations across the field of view. Since part of the patient may be within the bore of a scanner, this part is under shadow, while other parts of the body are illuminated by the ambient light. Variations in intensity cause difficulties in automated analysis of the images. This is in part also caused by the fact that only ambient illumination is available. This naturally illuminates the CT acquisition zone inside the bore much less than the rest of the scene.

Furthermore, for best performance, in some cases, camera and lenses with a very wide FOV may be chosen to cover the wide motion range of the patient table. Such optical systems are subject to strong geometrical distortions, which also induce strong brightness variation across the image since light from large solid angles is projected into relatively few pixels towards the rim of the image. This can lead to saturation of the raw image in these areas while the central image region inside the bore is poorly illuminated. It is particularly demanding to detect sub-millimeter respiratory motion of a patient while moving on a table through these image regions with strong intensity variations and geometrical distortions.

In view of the above challenges, it is the recognition of the inventors that improved motion and respiration tracking may be achieved by providing a specialized lighting arrangement which can help to balance the strong intensity variations.

The invention is defined by the claims.

According to examples in accordance with an aspect of the invention, there is provided a system comprising a medical imaging unit comprising a housing and an imaging apparatus, wherein the housing comprises a bore for receiving an object to be imaged, and wherein the imaging apparatus is for probing an imaging zone within the bore, and wherein an axial axis of the bore defines a z-direction. The system further comprises a lighting apparatus for providing illumination inside the bore of the imaging unit. The lighting apparatus comprises one or more light output areas disposed inside the bore of the imaging unit.

In at least one advantageous set of embodiments, the lighting apparatus comprises a plurality of independently controllable light output areas disposed inside the bore of the imaging unit, the plurality of light output areas being spaced from one another in the z-direction.

Thus, in accordance with at least one set of embodiments, it is proposed to provide a lighting apparatus with a plurality of axially spaced light outputs, thereby enabling provision of a spatially resolved light output across the z-axis of the bore. This advantageously facilitates the provision of an overall light output which is maximally homogeneous.

Preferably the light outputs are area light sources rather than point light sources. This is based on the insight that any standard point light source may introduce shadows to the scene which may undermine the purpose of maximizing homogeneity.

The system may further comprise a lighting controller adapted to implement a lighting control operation comprising individually controlling a setting of at least one light output property of each of the light output areas so as to form a controlled light intensity distribution as a function of z-position in the bore.

Thus, in this set of embodiments, a lighting controller may implement a control operation to provide a defined light intensity distribution across said z-axis direction. This aids in homogenizing the light distribution across the bore, enabling light conditions to be made more consistent, regardless of variable ambient light conditions. By providing each light output area with a controllable light output property, this provides the controller a degree of freedom in configuring homogeneity across an area or space in that light output property.

This is especially valuable in the context of systems employing patient observation cameras, for example for use in monitoring patient vital signs or patient position or patient motion, where homogenous light conditions across a visible surface of the patient is important.

The controlled light intensity distribution may be formed across a defined plane within the bore. For example, the plane may be a plane may be defined by an upper surface of a patient support table which is axially moveable through the bore in the z-direction.

The lighting apparatus may comprise a respective set of one or more light sources arranged for generating light output at each of the plurality of light output areas.

In some embodiments, the at least one light output property may include a brightness level of each of the light output areas. Additionally or alternatively, in some embodiments, the at least one light output property includes a color of the light output of each of the light output areas. In some embodiments, as discussed further below, the at least one light output property for each light output area may include a brightness level of each of a plurality of color channels of the light output area. For example, where each light output area provides an RGB light output, having a composite color formed from the combination of particular levels of each of red, green and blue channels, then the at least one light output property comprises an adjustable brightness or intensity of each of the red, green and blue channels.

In some embodiments, each of the one or more light output areas may comprise at least one light output strip extending part or all of the way around an interior circumference of the bore. The strip may for example be arcuate or annular in shape. This arrangement effectively provides at least one light arc or ring in the bore, for example a plurality of spaced light arcs or rings. By providing the light output area extended in the circumferential direction, this aids in providing a homogeneous light distribution since it avoids creation of shadows at lateral edges of the light output projection.

In some embodiments, the lighting apparatus includes at least one light diffusion panel having a light output surface, and wherein at least one of the light output areas is formed by at least a portion of the light output surface of the at least one light diffusion panel. A diffusion panel helps in providing a homogeneous light distribution.

Where a plurality of light output areas are to be provided, one option is to provide one diffusion panel with a plurality of spatially discrete light output areas generated at a light output surface of the panel. A further option is to provide a plurality of diffusion panels, wherein each light output area of the lighting apparatus is provided by a different one of the diffusion panels.

In some embodiments, each of two or more of the plurality of light output areas is formed by a respective area of the light output surface of the at least one light diffusion panel. Here, a plurality of spatially discrete light output areas are generated at a light output surface of a single diffusion panel.

In some embodiments, the light diffusion panel has a front light output face and a rear face opposite the front light output face, and wherein the lighting apparatus comprises a plurality of light sources arranged to couple light into the light diffusion panel through the rear face in order to form the plurality of light output areas at the front light output face. Here, patches of light on the single light diffusion panel form the different light output areas, coupled in from behind.

In some embodiments, the plurality of light sources comprises a plurality of OLED light sources.

In some embodiments, the lighting apparatus comprises one or more light sources arranged to couple light into edges of the light diffusion panel for transmission across the panel. In other words, light is coupled in from the side. This provides a structurally efficient configuration.

In some embodiments, the lighting control operation is configured such that the light intensity distribution is homogeneous in said at least one light output property across a pre-determined reference plane extending across the bore, e.g. the plane occupied by the patient table during scanning.

In accordance with one advantageous set of embodiments, it is proposed to make use of the arcuate x-ray cover which extends around an interior face of the bore in a CT imaging apparatus to provide a secondary function of emitting light.

In particular, in accordance with one set of embodiments, the medical imaging unit is a tomographic, e.g. computed tomography, CT, imaging unit comprising a gantry for carrying an x-ray generator and x-ray detector for probing the imaging zone, wherein an axial axis of the bore defines a z-direction. The CT imaging unit comprises an x-ray input/output window extending circumferentially around at least a portion of an interior face of the bore for facilitating x-ray communication between the imaging zone and an x-ray generator and detector carried by the gantry. At least one light output area may be formed by at least a portion of an exterior face of the x-ray input/output window.

Thus, here, the x-ray window is efficiently utilized both for light diffusion and x-ray communication with the imaging zone. This is structurally simple and avoids a need to add additional structural components inside the bore to provide the light output, which may impede functionality. This also has the advantage that the addition of any light sources should preferably entail only minimal changes to the design of the inside of the CT bore. In particular, the free bore width is not reduced.

The aforementioned x-ray input/output window may comprise a light diffusion panel. In other words, it is proposed here to replace the cover which covers the CT acquisition zone with a light diffusing cover. Light is coupled into the diffusing cover to provide an area light source.

It is therefore proposed in accordance with this set of embodiments to introduce a distributed diffuse light source to the CT bore. It is proposed to transform the existing cylindrical/a acrylic glass cover window of the CT acquisition zone into a light source to avoid any major required change to the inside of the bore. In some embodiments, light may be coupled into the light diffusion panel from side edges. In some embodiments, it is proposed to provide the diffusion panel with a roughened or frosted surface for providing a diffuse output.

It is noted that the concept of replacing the x-ray input/output window with a light diffusing panel which serves the dual function of x-ray input/output window and a diffusive light output area can be utilized with only a single light output area or multiple light output areas. For example, the entire panel may be illuminated to thereby provide a single homogeneous diffuse light output area, or multiple light output areas could be provided, for example by illuminating different patches or regions of the panel, in the manner already discussed above for example.

In accordance with a further advantageous set of embodiments, it is proposed to provide an intelligent control operation for the lighting apparatus which aims to provide a substantially homogenous light distribution by the combined light outputs of a plurality of light output areas.

Thus, in accordance with this set of embodiments, there is provided a lighting controller adapted to implement a lighting control operation comprising individually controlling a setting of at least one light output property of each of the light output areas so as to form a controlled light intensity distribution as a function of z-position in the bore.

The lighting control operation may comprise identifying for each of a plurality of points, x, across a field of interest (ROI):

an ambient light contribution, $P_0(x)$, at the point, and

a light contribution, $a_iP_i(x)$, at the point provided by each of the plurality of light output areas, i, as a function of the at least one controllable setting, $a_i$, of a light output property of the light output area.

Each light contribution, $a_iP_i(x)$, includes at least a measured value of the at least one light output property at the relevant point.

Using this information, the lighting control operation may comprise running an optimization procedure for fitting values of the setting, $a_i$, of the light output property for each of the light output areas. The optimization procedure may be configured for maximizing homogeneity in the light output property across the field of interest, wherein a value of the light output property at each point comprises a sum of the ambient light contribution, $P_0(x)$, at that point and the light contributions, $a_iP_i(x)$, by the plurality of light output areas.

With regards to the "light contribution, $a_iP_i(x)$, at the point provided by each of the plurality of light output areas, i, as a function of the at least one controllable setting, $a_i$, of a light output property of the light output area", this may in some embodiments be identified by measuring a light contribution, $P_i(x)$, at the point by each of the plurality of light output areas, i, at a maximum setting for the light output property, and wherein the setting $a_i$ is a controllable fractional weighting of said maximum.

As explained below, the field of interest may be a region of interest of an imaging field of view of a camera, and measurement of the light contributions at each point in the field of interest may be performed based on a received image having a field of view which includes the field of interest. The field of interest may be 2D, in the case of a standard 2D camera.

In some embodiments, the optimization procedure comprises:

defining a target value, $a_0(x)$, for the light output property for each point, x, of the field of interest, wherein preferably the target value is the same for every point, x; and

fitting the values of the controllable setting, $a_i$, of the light output property for each light output area so as to minimize, for each point x, a difference measure between the target value, $a_0$, and a sum of the ambient light contribution at that point, $P_0(x)$, and the aggregate (i.e. sum total) of the light contributions, $a_iP_i(x)$, provided by each of the plurality of light output areas at that point as a function of the setting, $a_i$.

For ensuring homogeneity, optionally, $a_0(x)$ is the same for every point, x, in which case it might just be referred to as $a_0$.

The field of points, x, may be one dimensional, e.g. in the z-direction.

Alternatively, the field of points may be two or three dimensional (the latter in the case of depth cameras), so that x may be a vector.

In some embodiments, the difference measure is a mean square difference.

In some embodiments, the optimization procedure may comprises performing the following minimization:

$$\min_{a_i \leq 1;\ i \in \mathbb{N}_0} \sum_{x \in ROI} \left( a_0(x) - \left[ P_0(x) + \sum_i a_i P_i(x) \right] \right)^2$$

where x is an index of the point in the field of interest,

$a_0(x)$ is a target value for the light output property for each point, x,

$P_0(x)$ is the ambient light contribution at each point, x,

$P_i(x)$ is the light contribution at each point, x, by each light output area, i, when the light output area is operated at a maximum value ($a_i = 1$) of the controllable setting of the light output property, and

$a_i$ is a fitting parameter representing a value of the controllable setting of the light output property of the light output area, i, where $a_i$ is a fractional weighting $0 \leq a_i \leq 1$.

For ensuring homogeneity, preferably, $a_0(x)$ is the same for every point, x, in which case it might just be referred to as $a_0$.

The parameter $a_0$ may be set equal to a level of ambient light outside of the bore such that there is homogeneity of light inside and outside the bore and so that there is no sharp step change in light level between inside and outside of the bore. This improves image quality. In addition, the value of $a_0$ may be determined so as to be greater than a spatial average over x within the bore of the values of $P_0(x)$ because the bore illumination sources can only add light and not subtract it.

If the ambient light level at the front end of the bore differs from that at the back end of the bore, then a non-constant function $a_0(x)$ may be chosen that provides a smooth transition of light level from front to back.

The at least one light output property may comprise a light intensity and/or a light color.

In some embodiments, each light output area may have two or more independently controllable light output property settings, and wherein the optimization procedure is run separately for each light output property.

For example, an RGB color of each light output area is adjustable per RGB channel and wherein each color channel is optimized and adjusted separately.

The system may comprise at least one camera for imaging a patient and having a field of view which spans at least the imaging zone within the imaging unit bore. The aforementioned field of interest may be at least a sub-zone of the field of view of the camera. The light contributions, $P_0(x)$, $P_i(x)$, at each point of the field of interest may be derived based on one or more images obtained with the camera.

The camera may be a monochromatic camera, or may be a color (e.g. RGB) camera.

In some embodiments, the optimization procedure may comprise receiving an ambient reference image generated by a camera having a field of view which spans at least the imaging zone within the imaging unit bore. The ambient reference image may depict the field of view with each of the plurality of light output areas deactivated, and wherein the ambient light contribution, $P_0(x)$, at each of the plurality of points, x, across the field of interest is derived from pixel values of said ambient reference image.

In some embodiments, the optimization procedure may comprise receiving, for each of the light output areas, a respective reference image generated by a camera having a field of view which spans at least the imaging zone within the imaging unit bore. Each of these reference images may depict the field of view with a single respective one of each of the plurality of light output areas activated at a maximum value ($a_i = 1$) of the controllable setting of the light output property, and wherein the light contribution, $P_i(x)$, at each of the plurality of points, x, across the field of interest for each light output area, i, is derived from pixel values of said reference images.

In some embodiments, the ambient light may have adjustable settings or modes or levels, and wherein the optimization is run separately for each of the multiple possible ambient lighting modes.

In accordance with any of the embodiments discussed above, the system may further comprise at least one camera for imaging a patient and having a field of view which spans at least the imaging zone within the gantry bore.

The camera may be a depth camera in specific embodiments e.g. an HR depth camera.

In some embodiments, the system comprises at least two cameras, one arranged one arranged for imaging each respective end of the imaging unit bore.

In some embodiments, the medical imaging unit is a CT or PET-CT, imaging unit comprising a gantry for carrying an x-ray generator and x-ray detector for probing the imaging zone, wherein an axial axis of the bore defines a z-direction.

Alternatively, embodiments of the invention are also applicable to magnetic resonance imaging devices.

Another aspect of the invention is a system comprising: a medical imaging unit comprising a housing and an imaging apparatus, wherein the housing comprises a bore for receiving an object to be imaged, and wherein the imaging apparatus is for probing an imaging zone within the bore, and wherein an axial axis of the bore defines a z-direction;

a lighting apparatus for providing illumination inside the bore of the imaging unit, the lighting apparatus comprising at least one light output area disposed inside the bore of the imaging unit;

wherein the medical imaging unit is a tomographic, e.g. computed tomography, CT, imaging unit comprising a gantry for carrying an x-ray generator and x-ray detector for probing the imaging zone;

wherein the CT imaging unit comprises an x-ray input/output window extending circumferentially around at least a portion of an interior face of the bore for facilitating x-ray communication between the imaging zone and an x-ray generator and detector carried by the gantry; and

wherein the at least one light output area is formed by at least a portion of an exterior face of the x-ray input/output window.

Any of the feature or embodiments described in relation to any other aspect of the invention can also be applied to this aspect of the invention.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a schematic illustration of an example system in accordance with one or more embodiments;
Fig. 2 schematically illustrates the light output fields of a set of light output areas of the system of Fig. 1;
Fig. 3 schematically illustrates sample positions and extensions of an example set of arcuate or annular light output areas in accordance with one or more embodiments;
Fig. 4 schematically illustrates the placement of an x-ray input/output window of a CT imaging unit in the place of which may be integrated a light diffusion panel in accordance with one or more embodiments;
Fig. 5 shows a model representation of an example area of interest across which an optimization algorithm may be employed to target a homogeneous composite light distribution formed from the individual light contributions of a set of light output areas; and
Fig. 6 schematically illustrates a frame of a captured image of a scene which includes a lighting apparatus in accordance with one or more embodiments, as may be used as a reference image as part of the optimization algorithm.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system comprising a medical imaging apparatus having an imaging zone defined by an interior region of a bore, and further comprising a lighting apparatus comprising one or more light output areas disposed within the bore. In one set of embodiments, there are provided a plurality of light output areas, distributed at different positions along an axial direction of the bore .In some embodiments, a controller is included to control a light output setting of each of the plurality of light output areas so as to form a pre-defined spatial light distribution from a composite of the light outputs of the plurality of light output areas. In some embodiments, an x-ray input/output window is replaced with an x-ray transmissive and light diffusive panel for performing dual functions of permitting x-ray communication between an imaging apparatus and the imaging zone and forming at least one wide-area diffuse/homogenous light output area.

As discussed above, in certain kinds of medical imaging, such as CT, MRI, or PET, it is advantageous to have capacity to monitor one or more physiological parameters of a patient in a contact-free manner. This can be done by mounting one or more cameras arranged to image a field of view which includes at least a region of the imaging zone, for capturing external, e.g. optical, images of the patient during the primary medical imaging operation. These images can be processed by algorithms to detect motion patterns or other features of the images and extract physiological parameters therefrom.

Respiratory monitoring involves the detection of sub-millimeter motion of the abdomen and chest. Monitoring of voluntary head motion requires a similar resolution. PPG cardiac monitoring is based on relative color and brightness changes in the order of 0.1%.

With reference to CT imaging, all types of camera-based monitoring pose challenges in view of the fact that CT acquisition is frequently performed with a moving patient table. This means that any observed body part is subject to brightness and color changes.

Moreover, in some cases, a camera and lenses are utilized having a very wide field of view, in order to cover the whole possible range of motion of the patient table. Such optical systems are subject to strong geometrical distortions, which also induce strong brightness variation across the image due to the light a from large solid angle being projected onto relatively few pixels towards the rim of the image. This can lead to saturation of the raw image in these areas while the image region inside the bore is poorly illuminated. This complicates motion monitoring of body parts, especially in this dark zone. However, it is in the region inside the bore where motion monitoring is most valuable, since this is where the CT or MRI or PET data is being acquired. Moreover, it is particularly demanding to detect small respiratory or head motions of a patient while moving on a table through these image regions with strong intensity variations and geometrical distortions.

These problems of intensity and color variations across the extent of the patient body arise in part due to the fact that the only light in the imaging room is typically ambient light, and that the ambient light is not configured for providing homogenous illumination, even outside of the bore. Inside of the bore, the ambient light typically only dimly penetrates, with the region mostly in shadow.

It is thus proposed in accordance with one or more embodiments to provide one or more sources of illumination within the bore itself.

One way to do this would be to provide one or more point-light sources in the CT bore. However, in preferred embodiments, there are instead provided one or more area light sources, referred to as light output areas in the forthcoming discussion. The optical source of illumination of these areas may itself be one or more point light sources, but at the light output surface exposed to the bore, there is preferably provided an area light output.

This is preferred to point light sources for the following reasons. First, point light sources, even a plurality of such, would not necessarily result in a homogenous illumination. Second, use of point light sources may introduce shadows to the scene which would add complication to camera-based monitoring, particularly in a moving table setting. Third, point light sources may in fact be hazardous to the patient who may be staring up at the roof of the bore during the imaging, and would be dazzled or blinded by the bright point sources.

In accordance with one set of embodiments discussed below, it is proposed to provide diffuse illumination by one or more large light-emitting areas of the bore wall. This may be implemented in accordance with one advantageous set of embodiments by using the x-ray transmission cover which is already integrated in the bore wall of CT scanners as the diffuse light emitter. Additionally or alternatively, in accordance with one or more embodiments, it is proposed to include within the bore region a plurality of light output areas which are distributed spaced relative to one another along the axial dimension of the bore. This assists in provision of a light distribution across the bore which is homogenous. Optionally, in accordance with one or more embodiments, it is proposed to provide a controller configured for independently controlling the light output areas to form a pre-determined light distribution across a region.

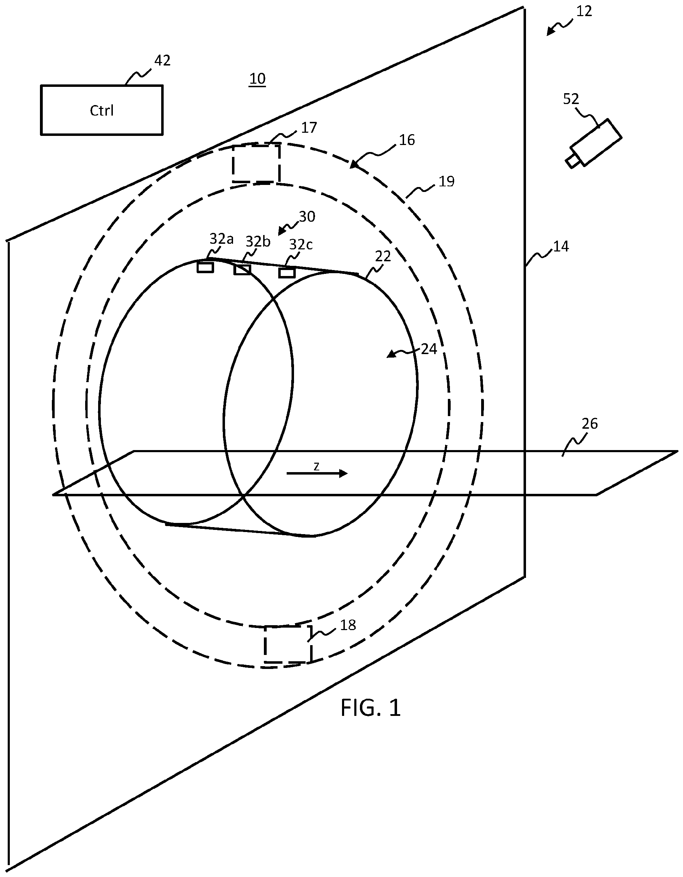

Fig. 1 schematically illustrates an example system 10 in accordance with one or more embodiments of the invention.

The system comprises a medical imaging unit 12 comprising a housing 14 and an imaging apparatus 16, 17, 18. The imaging apparatus may be accommodated in the housing. The housing comprises a bore 22 for receiving an object to be imaged. The imaging apparatus 16, 17, 18 is for probing an imaging zone 24 within the bore. An axial axis of the bore defines a z-direction.

There is further provided a lighting apparatus 30 for providing illumination inside the bore 22 of the imaging unit 12. In the illustrated example, the lighting apparatus comprises a plurality of independently controllable light output areas 32a, 32b, 32c disposed inside the bore of the imaging unit. The plurality of light output areas are spaced from one another in the z-direction, i.e. the axial dimension of the bore. Instead, just one light output area might be provided in accordance with a further set of embodiments.

In the present example, there is further provided a lighting controller 42 adapted to implement a lighting control operation comprising individually controlling a setting of at least one light output property of each of the light output areas 32a, 32b, 32c so as to form a controlled light intensity distribution as a function of z-position in the bore 22. For example the light intensity distribution might be formed across a planar area defined by an upper surface of patient table 26 of the system.

With regards to the light output areas 32a, 32b, 32c, as will be explained below, these may each comprise one or more light sources, for originating the optical output of the area, and comprise one or more optical components, e.g. light processing elements, for conditioning a light output from the area. Each may for example comprise a light output surface which is diffusive.

With regards to the controller, this may be operatively coupled with each of the light output areas, for example to the light sources comprised by each light output area. This may be a wired or wireless connection. In the schematic drawing of Fig. 1, the connection between the controller and the light output areas is not shown, for the sake of simplicity.

It is noted that although each of the light output areas 32a, 32b, 32c is shown spanning only a small area, this is purely for ease of graphical representation in this simplified diagram. In practical embodiments, each light output area may span a wider area, as will be explained shortly.

Accordingly, in accordance with one or more embodiments, it is proposed to introduce a set of, e.g. custom-designed and adjustable, light sources to the bore 22 of the medical imaging unit 12 to assist in compensating the strong intensity and color variations which can arise during imaging, particularly, e.g. when moving a patient from the zone dominated by ambient lighting into the bore 22. There is further proposed a system and method for adjustment of a light output property, e.g. the light levels, of the various light sources to minimize changes in illumination during a table move.

In the illustrated example, the medical imaging unit 12 is a computed tomography (CT), imaging unit comprising a gantry 16 for carrying an x-ray generator 17 and x-ray detector 18 for probing the imaging zone 24, and wherein an axial axis of the bore 22 defines a z-direction.

However, the principles of the invention are applicable equally to other imaging modalities, such as for example: magnetic resonance (MR) imaging, positron emission tomography (PET), and single-photon emission computed tomography (SPECT).

The system may further comprise at least one camera 52 for imaging a patient and having a field of view which spans at least the imaging zone 24 within the imaging unit bore.

The camera may be a black and white camera or a color camera. The camera may be a depth camera, e.g. an HR depth camera. Although not shown in Fig. 1, in some embodiments, the system comprises at least two cameras, one arranged for imaging each respective end of the imaging unit bore.

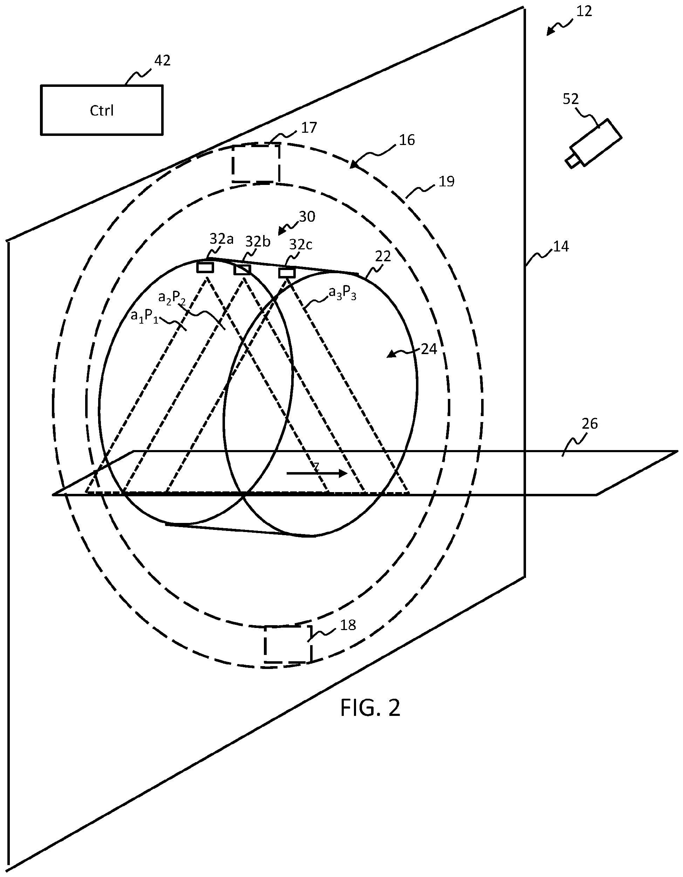

For illustration, Fig. 2 shows a schematic representation of example light outputs of the first 32a, second 32b, and third 32c light output areas of the lighting apparatus 30. These are shown in highly schematic form only, for purposes of illustrating the concept that the light output areas may each provide a respective illumination field which spans at least a portion of the interior of the bore 22 and wherein these illumination fields may overlap partially, thereby providing a means for constructing a composite light distribution field, e.g. across the whole area spanned by the patient table 26, by tuning the controllable light output property of each of the light output areas.

By way of a simple example, for the depicted illustration, each light output area 32a, 32b, 32c is assumed to have a single controllable light output property, wherein a controllable setting, $a$, of that light output property for light output area, i, is represented as $a_i$.

The light contribution provided by each light output area, i, is represented as $a_iP_i$, where $P_i$ represents light contribution, $P_i$, by each light output area, i, at a maximum setting for the light output property, and wherein the setting $a_i$ is taken to be a controllable fractional weighting of said maximum.

In the illustrated example, just three light output areas 32a, 32b, 32c are depicted. However, in practice, more or fewer may be provided.

The at least one controllable light output property of each light output area 32 may include a brightness level of each of the light output areas. Additionally or alternatively, the at least one controllable light output property of each light output area 32 may include a color of the light output of each of the light output areas.

In the schematic illustration of Fig. 1 and Fig. 2, the light output areas 32a, 32b, 32c are each shown as each spanning only a small area. However, this is purely for ease of graphical representation. In practical embodiments, each light output area may span a wider area.

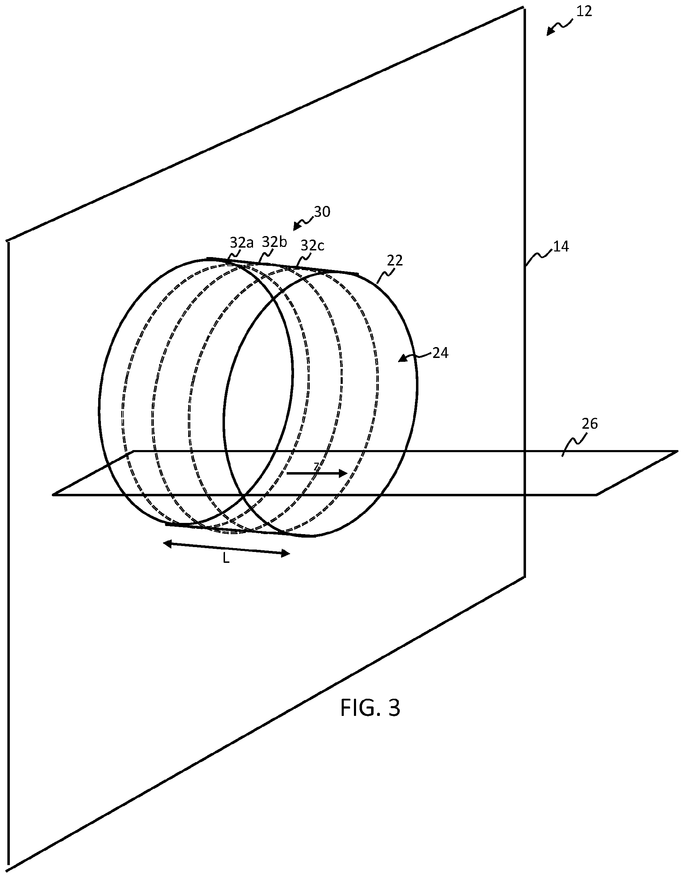

For example, each of the light output areas 32a, 32b, 32c may comprises at least one light output strip extending part or all of the way around an interior circumference of the bore. The strip may be arcuate or annular in shape. This arrangement effectively provides at least one light arc in the bore, for example a plurality of spaced light arcs or rings. This is illustrated schematically in Fig. 3, where the arcuate extensions of each of the light output areas 32a, 32b, 32c are indicated by dashed lines. The output area of each light output area may be wider in the z-direction than is depicted in this schematic representation, such that each light output area may form an arcuate or annular strip for example. The light output areas may extend the whole way or substantially the whole way around the circumference, so as to form light rings. The light output areas may just extend around the portion of the bore circumference which is vertically above the patient table.

To form these light output strips, each may comprise a respective light output surface which is diffusive. One or more light sources may be arranged to couple light into each light output surface, for example LEDs. Standard LED technology can be used for the proposed system, but slim-line LED strips may be used for minimal reduction of bore diameter. Although it is not essential, the arcuate light output strips may be placed mirror-symmetrically to a center of the CT bore along the z-direction (= table move direction). For example, if z= 0 represents the center position of the bore such that both ends of the bore are located at z=-L/2 and z = L/2 with L = bore length, then N twin pairs of LED rings may be located at positions z= - $d_i$ and z = $d_i$ with $d_i$ being the distances from the center of the bore and index i=1..N. A central ring may also be present at z=0. Brightness and/or color of each ring may be adjustable.

The use of arcuate light output areas is just one example, and other shapes and extensions can also be used.

It is further noted that, in the schematic illustration of Fig. 1, each light output area 32a, 32b, 32c is shown as being emitted from a discrete unit. Again, this is for ease of representation only. In some embodiments, there may be incorporated a light diffusing panel, and wherein each light output area is formed as a respective area of the light diffusing panel. For example, with reference to the light strips referred to above, instead of each light strip having a separate respective light output surface at the interior face of the bore, instead, each may be formed as a discrete light output area from a common unitary light emission surface.

In some embodiments, the lighting apparatus 30 includes at least one light diffusion panel having a light output surface, and wherein at least one of the light output areas 32a, 32b, 32c is formed by at least a portion of the light output surface of the at least one light diffusion panel.

One option is to provide one diffusion panel with a plurality of spatially discrete light areas generated at a light output surface of the panel. A further option is to provide a plurality of diffusion panels, wherein each light output area of the lighting apparatus is provided by a different one of the diffusion panels.

The previously mentioned arcs or rings may be formed as arcuate or annular sub-areas of the light diffusion panel, or multiple arcuate diffusion panels could be provided.

In a preferred embodiment, a single panel is provided.

For the light diffusion panel, this may comprise a curved sheet of material which is for example frosted or has a matte surface. It may be cylindrical in shape and extend the full circumference of the inner face of the bore. By way of one example, it may be formed of acrylic glass. Illumination might be realized by either coupling light into the side edges of the light diffusive panel, or by locating light sources at a rear face of the panel to couple light in from the rear side. Side-coupling of light may be preferred, particularly in CT and PET applications, since it may help to avoid any modulation of the inbore illumination due to the rotation of the gantry.

In some examples, the light sources for illuminating the light output areas 32a, 32b, 32c may be provided by one or more LED light strips, for example located at both edges of the light diffusion panel, preferably the full arcuate extension of the panel, to achieve homogenous illumination.

The degree of light diffusion, e.g. the amount of frosting, may be varied across the surface to achieve homogenous illumination, e.g., less frosting towards the edges, where the LED lighting is located, and more frosting toward the center, to achieve a greater scattering of light towards the central transverse plane of the sheet, to compensate for the larger distance to the LEDs.

With regards to the suitable light frequency for the light output areas, at least visible light or infrared (IR) light would be suitable. For example, if acrylic glass is used for the light output surface, the light absorption of this material is very low for both visible and infra-red (IR) light up to wavelengths of 1 100nm. The solution may be implemented with one or both light types. If IR light and IR cameras are used for respiratory monitoring, then the visible illumination can be independently adjusted as convenient for the patient.

As mentioned above, where a light diffusion panel is used, there are different options as to how to form the multiple light output areas. In some embodiments, each of two or more of the plurality of light output areas is formed by a respective area of the light output surface of the at least one light diffusion panel. Here, a plurality of spatially discrete light areas are generated at a light output surface of a single diffusion panel. These might otherwise be referred to as areal light sources.

The bore cover may be segmented into several zones, each used as an individually controllable light source. Patches of LEDs or OLEDs may be integrated for that purpose, or patches of transparent plastics with a frosted surface where light is coupled into the edges of the plastic layer and scattered out into the bore through the frosted surface.

The alternative option is to provide multiple light diffusion panels.

In some embodiments, a provided light diffusion panel may have a front light output face and a rear face opposite the front light output face, and wherein the lighting apparatus comprises a plurality of light sources arranged to couple light into the light diffusion panel through the rear face in order to form the plurality of light output areas at the front light output face.

Here, patches of light on the single light diffusion panel form the different light output areas, coupled in from behind. Additionally or alternatively, the lighting apparatus may comprise one or more light sources arranged to couple light into edges of the light diffusion panel for transmission across the panel. These may for example be LEDs such as OLEDs. In other words, in this case, light is coupled in from the side. This provides a structurally efficient configuration.

In any of these cases, preferably, the lighting control operation performed by the lighting controller 42 is configured such that the light intensity distribution is homogeneous in said at least one light output property across a pre-determined reference plane extending across the bore 24, e.g. the plane occupied by the patient table 26 during scanning.

As mentioned briefly above, in accordance with some embodiments it is proposed to adapt the x-ray window of the bore of CT scanner for a secondary use of light emission. This is structurally and functionally efficient. It is noted that this inventive concept provides technical benefits whether used with a single light output area or multiple light output areas.

In accordance with this set of embodiments, the medical imaging unit 12 is in particular a tomographic, e.g. computed tomography, CT, imaging unit comprising an imaging apparatus which comprises a gantry 16 for carrying an x-ray generator 17 and x-ray detector 18 for probing the imaging zone 24.

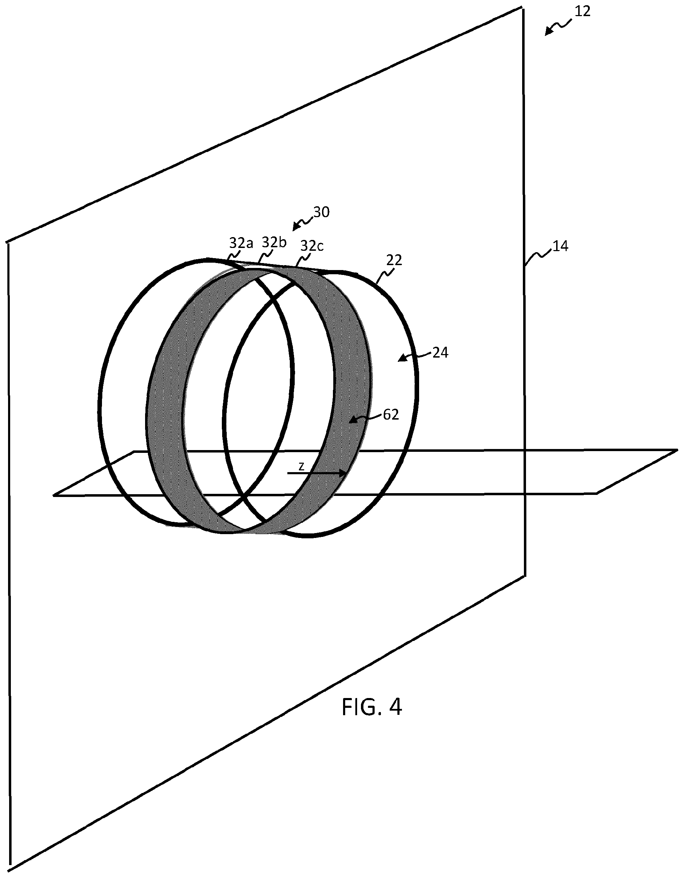

As schematically depicted in Fig. 4, the CT imaging unit 12 comprises an x-ray input/output window 62 extending circumferentially around at least a portion of an interior face of the bore 22 for facilitating x-ray communication between the imaging zone 24 and an x-ray generator 17 and detector 18 carried by the gantry 16. At least one of the plurality of light output areas is formed by at least a portion of an exterior face of the x-ray input/output window. Fig. 4 schematically indicates the location of the x-ray input/output window 62 relative to the bore.

Thus, in accordance with this set of embodiments, the x-ray window 62 is efficiently utilized both for light diffusion and x-ray communication with the imaging zone 24.

For example, in some embodiments, the x-ray input/output window 62 may be provided in the form of a light diffusion panel (which is also transparent to x-rays, or at least x-ray transmissive). Thus, the glass cover which covers the CT acquisition zone in certain state of the art CT scanners is replaced with a light diffusing cover (which is also x-ray transmissive). Light is coupled into the diffusing cover to provide an area light source.

In known CT scanners, clear cylindrical acrylic glass is used for the x-ray input/output window 62. The purpose of providing such a window is to reduce x-ray absorption en-route to the imaging zone. The remainder of the bore is typically formed of fiber-reinforced material which is more x-ray absorptive. Acrylic glass, unlike fiber-reinforced material, provides low and homogenous X-ray absorption.

Any of the various implementation options and features discussed above in relation to provision of a light diffusion panel may be applied to the joint x-ray window and light diffusion panel proposed in accordance with the present set of embodiments. These will therefore not be recited here to avoid repetition.

In addition to advantages of improved light homogeneity and thus improved camera-based patient monitoring, this set of embodiments of the invention also confers at least the following advantages.

Due to the diffusive material of the x-ray window, there is no longer visibility for the patient of the fast rotating gantry 16, which may reduce anxiety for patients.

Furthermore, by providing illumination inside the bore, this improves the experience for the user. Being located inside the bore can trigger feelings of anxiety and claustrophobia. The illuminated bore will appear wider to a human observer than a non-illuminated bore. This therefore reduces claustrophobia for patients.

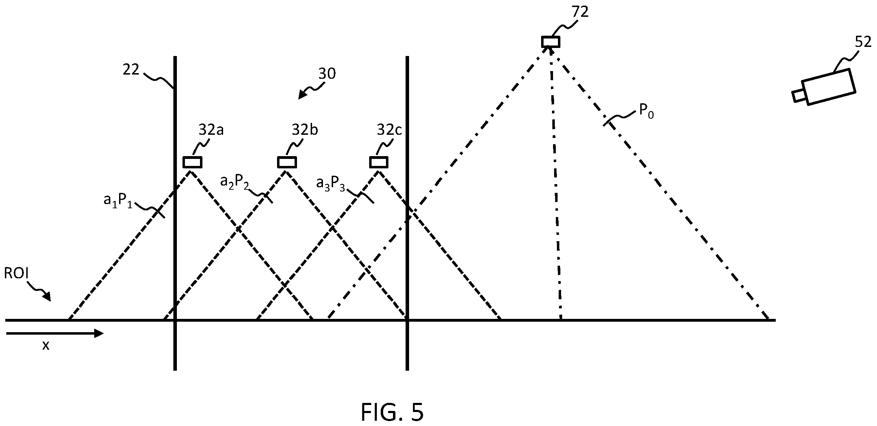

As briefly mentioned above, in accordance with one or more embodiments it is proposed to provide a plurality of light output areas in the bore, each with at least one controllable light output property, and to further provide an intelligent control operation in which the particular combination of settings for that light output property for the different light output areas is adjusted to form an overall light distribution which, in combination with the ambient light in the space, is substantially homogeneous. This involves application of an optimization routine in which light output parameters for each the plurality of light output areas are fitted, based on a target homogeneous value for the light output property, and based on knowledge of a light contribution by the ambient lighting and by each of the light output areas to each point across an area of interest. This will now be discussed in more detail, with reference to Fig. 5.

In summary, a light adjustment procedure is proposed. This may in some embodiments be performed for a given ambient lighting state, since it depends upon the ambient light contribution to each point across the field of interest. If there are several different possible ambient modes, the optimization procedure outlined below may be performed for each mode, for example during system installation or servicing. Resulting settings for the at least one light output property of each light output area (e.g. brightness and/or color) may be stored and the settings automatically applied when the respective ambient lighting is used by staff.

In some embodiments, the one or more bore cameras 52 may be used to detect the ambient lighting mode and adjust the settings of the light output areas accordingly. The bore light sources may be dimmed to detect this mode.

In the following example adjustment procedure, for simplicity, each light output area is assumed to have only a single available light output color, e.g. white, and furthermore the camera or cameras are assumed to be black and white cameras. Thus, in this case, each light output area has only a single adjustable property, namely brightness or intensity, and wherein a setting of this property is controllable for each light output area independently by the controller.

In alternative cases where the light output of each light output area is color-adjustable, i.e. a brightness is adjustable per channel (R,G,B) and RGB cameras are available, then the optimal brightness settings for each color channel may be optimized separately in accordance with the following procedure.

To assist understanding in the following discussion, Fig. 5 shows a schematic model representation of an example field of view, spanning dimension x. Illustrated are a plurality of light output areas 32a, 32b, 32c of the lighting apparatus 30, having the example form or structure in accordance with any of the options previously discussed, or otherwise. The outlines of the bore 22 are schematically indicated with vertical lines. Also schematically illustrated is an ambient light source 72. In this simplified model, this is taken to represent the totality of ambient light which impinges on the field of interest (ROI).

The light output of each light output area is schematically depicted. By way of a simple example, for the depicted illustration, each light output area 32a, 32b, 32c is assumed to have a single controllable light output property, wherein a controllable setting, $a$, of that light output property for light output area, i, is represented as $a_i$. The light contribution provided by each light output area, i, is represented as $a_i P_i$, where $P_i$ represents light contribution, $P_i$, by each light output area, $i$, at a maximum setting for the light output property, and wherein the setting $a_i$ is taken to be a controllable fractional weighting of said maximum. Three light output areas 32a, 32b, 32c are shown in this example, but any number i=1...N can be provided. The ambient light output is also represented, as $P_0$.

As schematically depicted, the various optical outputs of the different sources of light overlap and mix across the span of the field of interest (ROI). The field of interest is taken to be one-dimensional for this model.

The objective is to provide a homogenous value of the controllable light property (brightness in the present example) across the whole extent of the field of interest (ROI). To achieve this, the brightness settings of the different controllable light output areas 32a, 32b, 32c must be tuned such that when all the sources of light are mixed together, the resulting composite light field is as close as possible to homogeneous in brightness across the field of interest, x.

Homogeneity in brightness across the whole region spanning both inside and outside the bore 22 is advantageous since it means that there is no change in lighting as the patient moves from outside to inside the bore, which is particularly valuable in, e.g. CT imaging, where imaging is performed with a moving patient table.

To implement the optimization, and with reference to Fig. 5, the lighting control operation performed by the controller may comprise first identifying, for each of a plurality of points, x, across a field of interest (ROI):

an ambient light contribution, $P_0(x)$, at the point; and
a light contribution, $a_i P_i(x)$, at the point provided by each of the plurality of light output areas 32a, 32b, 32c, indexed by i, as a function of the at least one controllable setting, $a_i$, of a light output property of the light output area.

Each light contribution, $a_i P_i(x)$, includes at least a measured value of the at least one light output property at the relevant point. In the present example, the light output property may be understood to be brightness.

The control operation further comprises running an optimization procedure for fitting values of the setting, $a_i$, of the light output property for each of the light output areas 32a, 32b, 32c, the optimization procedure configured for maximizing homogeneity in the light output property across the field of interest ROI, wherein a value of the light output property at each point comprises a sum of the ambient light contribution, $P_0(x)$, at that point and the light contributions, $a_i P_i(x)$, by the plurality of light output areas.

With regards to the "light contribution, $a_i P_i(x)$, at the point provided by each of the plurality of light output areas, i, as a function of the at least one controllable setting, $a_i$, of a light output property of the light output area", this may for example be identified by measuring a light contribution, $Pi(x)$, at the point by each of the plurality of light output areas, i, at a maximum setting for the light output property, and wherein the setting ai is a controllable fractional weighting of said maximum.

As explained below, the field of interest may be a region of interest of an imaging field of view of a camera 52, and measurement of the light contributions at each point in the field of interest may be performed based on a received image having a field of view which includes the field of interest. The field of interest may be 2D, in the case of a standard 2D camera, or 3D in the case of a depth imaging camera.

In preferred embodiments, the optimization procedure may be performed just once, e.g. during initialization or installation, and the settings then stored for later recall.

To explain the optimization further, the optimization procedure may comprise: defining a target value, $a_0(x)$, for the light output property for each point, x, of the field of interest (ROI), and preferably wherein preferably the target value is the same for every point, x (i.e. to target homogeneity in the light output property).

The procedure may then further comprise fitting the values of the controllable setting, $a_i$, of the light output property for each light output area so as to minimize, for each point x, a difference measure between the target value, $a_0$, and a sum of the ambient light contribution at that point, $P_0(x)$, and the aggregate (sum total) of the light contributions, $a_i P_i(x)$, provided by each of the plurality of light output areas at that point, x, as a function of the setting, $a_i$.

The field of points, x, may be one dimensional.

Alternatively, the field of points may be two or three dimensional (the latter in the case of depth cameras), so that x may be a vector.

In some embodiments, the difference measure may be a mean square difference. However, any other suitable difference measure could be used. For example, simply a difference of the absolute values may suffice.

To give a more detailed example, in some embodiments, optimization procedure may comprises performing the following minimization:

$$\min_{a_i \leq 1;\ i \in \mathbb{N}_0} \sum_{x \in ROI} \left( a_0(x) - \left[ P_0(x) + \sum_i a_i P_i(x) \right] \right)^2$$

where x is an index of the point in the field of interest,

$a_0(x)$ is a target function for the light output property for each point, x,

$P_0(x)$ is the ambient light contribution at each point, x,

$P_i(x)$ is the light contribution at each point, x, by each light output area, i, when the light output area is operated at a maximum value ($a_i$ = 1) of the controllable setting of the light output property, and

$a_i$ is a fitting parameter representing a value of the controllable setting of the light output property of the light output area, i, where $a_i$ is a fractional weighting $0 \leq a_i \leq 1$.

For ensuring homogeneity, preferably, $a_0(x)$ is the same for every point, x, in which case it might just be referred to as $a_0$.

In some embodiments, the parameter $a_0$ may be set equal to a level of ambient light outside of the bore such that there is homogeneity of light inside and outside the bore and so that there is no sharp step change in light level between inside and outside of the bore. This improves image quality. In addition, the value of $a_0$ may be determined so as to be greater than a spatial average over x within the bore of the values of $P_0(x)$ because the bore illumination sources can only add light and not subtract it.

If the ambient light level at the front end of the bore differs from that at the back end of the bore, then a non-constant function $a_0(x)$ may be chosen that provides a smooth transition of light level from front to back.

As discussed above, in some embodiments, the at least one light output property comprises a light intensity. It may consist only of light intensity. Additionally or alternatively, it may comprise a light color, in which case the optimization discussed above might be performed separately for each color channel..

In other words, in cases where each light output area has two or more independently controllable light output property settings, the optimization procedure may be run separately for each light output property.

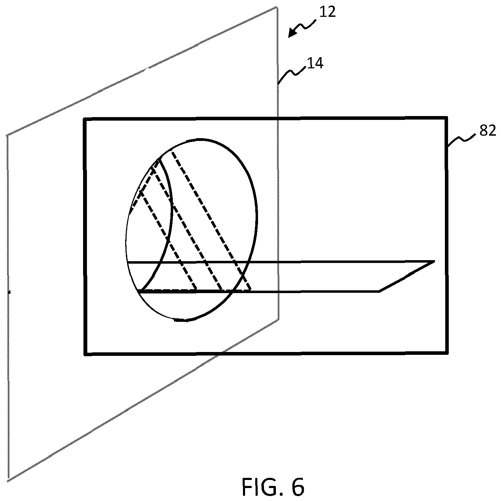

The model representation shown in Fig. 5 is to be understood as a demonstration of the concepts, rather than a geometrically accurate representation of the region of interest. In particular, in most embodiments, the points of the region of interest may simply be taken as the pixels of a reference image acquired by the relevant camera 52, with a camera field of view which includes at least a section of an interior of the bore. Purely for illustration, Fig. 6 shows an outline 82 of an example camera image which might be captured of the overall scene, and wherein this includes within its image plane only a portion of the interior of the bore being visible.

Thus, although Fig. 5 shows the region of interest points, x, spanning a linear line along the z-direction of the bore, in reality, the x-points may simply span a dimension of the rows and/or columns pixels of a captured image 82. However, exactly the same computation can be applied, since each pixel in the image will have an ambient light contribution, and a light contribution from each of the light output areas 32a, 32b, 32c, and the object is in fact to provide homogenous illumination across a field of view of the camera itself. Therefore it makes sense to perform the optimization with respect to a light field which is visible from the point of view of a particular camera. Thus, where there is more than one camera, the optimization may be performed separately for each camera.

To put this in more concrete terms, in some embodiments, the system comprises at least one camera 52 for imaging a patient and having a field of view which spans at least the imaging zone 24 within the imaging unit bore 22, and wherein the field of interest, ROI, is at least a sub-zone of the field of view of the camera, and wherein the light contributions, $P_0(x)$, $P_i(x)$, at each point of the field of interest are derived based on one or more images obtained with the camera. For example, the different points, x, may correspond to pixels of the image field of view of the camera.

The camera may be a monochromatic camera, or may be a color (e.g. RGB) camera.

To derive the values for $P_0$ and $P_1$, $P_2$, $P_3$ mentioned above, a set of reference images may be obtained using the camera as follows.

In some embodiments, the optimization procedure may comprise receiving an ambient reference image generated by a camera having a field of view which spans at least the imaging zone 24 within the imaging unit bore 22, and wherein the ambient reference image depicts the field of view with each of the plurality of light output areas 32a, 32b, 32c deactivated, and wherein the ambient light contribution, $P_0(x)$, at each of the plurality of points, x, across the field of interest is derived from pixel values of said ambient reference image.

In addition to this, the optimization procedure may comprise receiving, for each of the light output areas, a respective reference image generated by a camera having a field of view which spans at least the imaging zone 24 within the imaging unit bore 22, wherein each reference image depicts the field of view with a single respective one of each of the plurality of light output areas 32a, 32b, 32c activated at a maximum value ($a_i$ = 1) of the controllable setting of the light output property, and wherein the light contribution, $P_i(x)$, at each of the plurality of points, x, across the field of interest for each light output area, i, is derived from pixel values of said reference images.

As mentioned above, in some embodiments, the ambient light may have adjustable settings or modes or levels, and wherein the optimization is run separately for each of the multiple possible ambient lighting modes.

In addition to advantages of improved light homogeneity and thus improved camera-based patient monitoring, embodiments of the invention also confers the following advantages.

By providing illumination inside the bore, this improves the experience for the user. Being located inside the bore can trigger feelings of anxiety and claustrophobia. The illuminated bore will appear wider to a human observer than a non-illuminated bore. This therefore reduces claustrophobia for patients.

A further advantage is that, where illumination with variable color tone is provided, this might be coordinated with ambient lighting color to provide a pleasant, e.g. relaxing, overall experience for the patient and for an operator.

Embodiments discussed above include a medical imaging unit with an imaging apparatus. At minimum, there may simply be provided the housing 14 of the imaging unit 12, with a bore 22 defined therein, and with the lighting apparatus 30 incorporated in the bore. As will be appreciated, the core of the various embodiments resides in the lighting rather than the medical imaging operation itself. Nonetheless, in some embodiments, the medical imaging apparatus may further be provided also integrated in the housing. One example imaging modality which is applicable with embodiments of the present invention is a CT imaging apparatus.

However, the invention can also be applied to other imaging modalities, including but not limited to MRI and PET imaging.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

**1.** A system (10), comprising:

a medical imaging unit (12) comprising a housing (14) and an imaging apparatus (16, 17, 18), wherein the housing comprises a bore (22) for receiving an object to be imaged, and wherein the imaging apparatus is for probing an imaging zone (24) within the bore, and wherein an axial axis of the bore defines a z-direction;
a lighting apparatus (30) for providing illumination inside the bore (22) of the imaging unit, the lighting apparatus comprising a plurality of independently controllable light output areas (32a, 32b, 32c) disposed inside the bore of the imaging unit, the plurality of light output areas being spaced from one another in the z-direction;
a lighting controller (42) adapted to implement a lighting control operation comprising individually controlling a setting of at least one light output property of each of the light output areas (32a, 32b, 32c) so as to form a controlled light intensity distribution as a function of z-position in the bore.

2. The system of claim 1, wherein:

the at least one light output property includes a brightness level of each of the light output areas; and/or
the at least one light output property include a color of the light output of each of the light output areas.

3. The system of claim 1 or 2, wherein each of the light output areas comprises at least one light output strip extending part or all of the way around an interior circumference of the bore.

4. The system of any of claims 1-3, wherein the lighting apparatus includes at least one light diffusion panel having a light output surface, and wherein at least one of the light output areas is formed by at least a portion of the light output surface of the at least one light diffusion panel.

5. The system of claim 4, wherein each of two or more of the plurality of light output areas is formed by a respective area of the light output surface of the at least one light diffusion panel.

6. The system of claim 4 or 5, wherein the light diffusion panel has a front light output face and a rear face opposite the front light output face, and wherein the lighting apparatus comprises a plurality of light sources arranged to couple light into the light diffusion panel through the rear face in order to form the plurality of light output areas at the front light output face.

7. The system of claim 4, wherein the lighting apparatus comprises one or more light sources arranged to couple light into edges of the light diffusion panel for transmission across the panel.

8. The system of any of claims 1-7,

wherein the medical imaging unit is a tomographic, e.g. computed tomography, CT, imaging unit comprising a gantry for carrying an x-ray generator and x-ray detector for probing the imaging zone, wherein an axial axis of the bore defines a z-direction;
wherein the CT imaging unit comprises an x-ray input/output window extending circumferentially around at least a portion of an interior face of the bore for facilitating x-ray communication between the imaging zone and an x-ray generator and detector carried by the gantry; and
wherein at least one of the plurality of light output areas is formed by at least a portion of an exterior face of the x-ray input/output window, and
preferably wherein the x-ray input/output window comprises a light diffusion panel.

9. The system of any preceding claim, wherein the lighting control operation further comprises:
identifying for each of a plurality of points, x, across a field of interest (ROI):

an ambient light contribution, $P_0(x)$, at the point;
a light contribution, $a_i P_i(x)$, at the point provided by each of the plurality of light output areas, i, as a function of the at least one controllable setting, $a_i$, of a light output property of the light output area,
wherein each light contribution, $a_i P_i(x)$, includes at least a measured value of the at least one light output property at the relevant point; and
running an optimization procedure for fitting values of the setting, $a_i$, of the light output property for each of the light output areas, the optimization procedure configured for maximizing homogeneity in the light output property across the field of interest, wherein a value of the light output property at each point comprises a sum of the ambient light contribution, $P_0(x)$, at that point and the light contributions, $a_i P_i(x)$, by the plurality of light output areas.

10. The system of claim 9, wherein the optimization procedure comprises:

defining a target value, $a_0(x)$, for the light output property for each point, x, of the field of interest, wherein preferably the target value is the same for every point, x.

fitting the values of the controllable setting, $a_i$, of the light output property for each light output area so as to minimize, for each point x, a difference measure between the target value, $a_0(x)$, and a sum of the ambient light contribution at that point, $P_0(x)$, and the aggregate of the light contributions, $a_i P_i(x)$, provided by each of the plurality of light output areas at that point as a function of the setting, $a_i$.

11. The system of claim 9 or 10, wherein the optimization procedure comprises performing the following minimization:

$$\min_{a_i \leq 1;\ i \in \mathbb{N}_0} \sum_{x \in ROI} \left( a_0(x) - \left[ P_0(x) + \sum_i a_i P_i(x) \right] \right)^2$$

where x is an index of the point in the field of interest,
$a_0(x)$ is a target function for the light output property for each point, x,
$P_0(x)$ is the ambient light contribution at each point, x,
$P_i(x)$ is the light contribution at each point, x, by each light output area, i, when the light output area is operated at a maximum value ($a_i = 1$) of the controllable setting of the light output property, and
$a_i$ is a fitting parameter representing a value of the controllable setting of the light output property of the light output area, i, where $a_i$ is a fractional weighting $0 \leq a_i \leq 1$.

12. The system of any of claims 9-11, wherein each light output area has two or more independently controllable light output property settings, and wherein the optimization procedure is run separately for each light output property.

13. The system of any of claims 9-12,

wherein the system comprises at least one camera for imaging a patient and having a field of view which spans at least the imaging zone within the imaging unit bore,
wherein the field of interest is at least a sub-zone of the field of view of the camera,
wherein the light contributions, $P_0(x)$, $P_i(x)$, at each point of the field of interest are derived based on one or more images obtained with the camera.

14. The system of any of claim 13,

wherein the optimization procedure comprises receiving an ambient reference image generated by a camera having a field of view which spans at least the imaging zone within the imaging unit bore,
wherein the ambient reference image depicts the field of view with each of the plurality of light output areas deactivated, and
wherein the ambient light contribution, $P_0(x)$, at each of the plurality of points, x, across the field of interest is derived from pixel values of said ambient reference image; and
wherein the optimization procedure comprises receiving, for each of the light output areas, a respective reference image generated by a camera having a field of view which spans at least the imaging zone within the imaging unit bore,
wherein each reference image depicts the field of view with a single respective one of each of the plurality of light output areas activated at a maximum value ($a_i = 1$) of the controllable setting of the light output property, and
wherein the light contribution, $P_i(x)$, at each of the plurality of points, x, across the field of interest for each light output area, i, is derived from pixel values of said reference images.

15. The system of any preceding claim, further comprising at least one camera for imaging a patient and having a field of view which spans at least the imaging zone within the imaging unit bore, and optionally wherein the system comprises at least two cameras, one arranged for imaging each respective end of the imaging unit bore.

FIG. 1

42

Ctrl

12

52

17

16

19

14

30

32a 32b 32c

22

$a_1P_1$ $a_2P_2$ $a_3P_3$

24

26

z

18

FIG. 2

FIG. 3

FIG. 4

FIG. 5

FIG. 6

## EUROPEAN SEARCH REPORT

Europäisches
Patentamt
European
Patent Office
Office européen
des brevets

**Application Number**

EP 23 18 2105

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2008 043725 A (GE MED SYS GLOBAL TECH CO LLC) 28 February 2008 (2008-02-28) | 1-8,15 | INV. A61B5/00 |
| A | * abstract * * paragraphs [0076], [0077], [0081], [0085], [0088], [0089]; claim 9; figure 5 * | 9-14 | A61B6/03 A61B5/055 A61B5/024 A61B5/113 F21W131/20 |
| | ----- | | |
| X | EP 2 288 288 B1 (KONINKLIJKE PHILIPS NV [NL]) 28 November 2018 (2018-11-28) | 1-8,15 | |
| A | * abstract * * paragraphs [0001], [0017], [0021] - [0027]; figures 1,2,5A-B * & WO 2009/147608 A1 (KONINKL PHILIPS ELECTRONICS NV [NL]; HEROLD MARK DOUGLAS [US] ET AL.) 10 December 2009 (2009-12-10) | 9-14 | |
| | ----- | | |
| X | EP 3 501 393 A1 (ELEKTA LTD [GB]) 26 June 2019 (2019-06-26) | 1-8,15 | |
| A | * abstract * * paragraphs [0018], [0019], [0024], [0025], [0037], [0042] * | 9-14 | |
| | ----- | | TECHNICAL FIELDS SEARCHED (IPC) |
| A | EP 4 113 145 A1 (KONINKLIJKE PHILIPS NV [NL]) 4 January 2023 (2023-01-04) * abstract * * claims 1-6; figure 1 * * the whole document * | 1-15 | A61B F21W |
| | ----- | | |
| A | JP H05 146423 A (SHIMADZU CORP) 15 June 1993 (1993-06-15) * abstract * * claim 1; figure 2 * * the whole document * | 1-15 | |
| | ----- | | |

The present search report has been drawn up for all claims

| Place of search | Date of completion of the search | Examiner |
|---|---|---|
| The Hague | 8 December 2023 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................

& : member of the same patent family, corresponding document

EPO FORM 1503 03.82 (P04C01)

22

## ANNEX TO THE EUROPEAN SEARCH REPORT
## ON EUROPEAN PATENT APPLICATION NO.

EP 23 18 2105

This annex lists the patent family members relating to the patent documents cited in  the above-mentioned European search report.
The members are as contained in the European Patent Office EDP file on
The European Patent Office is in no way liable for these particulars which are merely given for the purpose of information.

08-12-2023

| Patent document cited in search report | | Publication date | Patent family member(s) | | Publication date |
|---|---|---|---|---|---|
| JP 2008043725 | A | 28-02-2008 | NONE | | |
| EP 2288288 | B1 | 28-11-2018 | CN | 102046084 A | 04-05-2011 |
| | | | EP | 2288288 A1 | 02-03-2011 |
| | | | US | 2011082348 A1 | 07-04-2011 |
| | | | WO | 2009147608 A1 | 10-12-2009 |
| EP 3501393 | A1 | 26-06-2019 | AU | 2018260851 A1 | 11-07-2019 |
| | | | CA | 3024045 A1 | 21-06-2019 |
| | | | CN | 110006013 A | 12-07-2019 |
| | | | EP | 3501393 A1 | 26-06-2019 |
| | | | GB | 2569797 A | 03-07-2019 |
| | | | US | 2019195487 A1 | 27-06-2019 |
| EP 4113145 | A1 | 04-01-2023 | EP | 4113145 A1 | 04-01-2023 |
| | | | WO | 2023274812 A1 | 05-01-2023 |
| JP H05146423 | A | 15-06-1993 | NONE | | |

EPO FORM P0459

For more details about this annex : see Official Journal of the European Patent Office, No. 12/82